# EUROPEAN PATENT APPLICATION

(11) **EP 3 636 234 A1**
(43) Date of publication of application: **15.04.2020**
(21) Application number: 18813083.5
(22) Date of filing: 31.05.2018
(51) Int. Cl.: A61F 13/15, A61F 13/42, A61F 13/44, A61F 13/511, G01N 33/52, A61F 13/51

(54) **METHOD FOR MANUFACTURING ABSORPTION PRODUCT**

(30) Priority: 05.06.2017 KR 20170069598; 28.02.2018 KR 20180024457; 28.02.2018 KR 20180024464
(71) Applicant: Craders Co.,Ltd, Seoul 02841 (KR)
(72) Inventor: LEE, Ui Cheol, Seoul 06241 (KR)
(74) Representative: Betten & Resch
(86) International application number: PCT/KR2018/006212
(87) International publication number: WO 2018/225977

(57) **Abstract**

A method for manufacturing an absorption product of the present disclosure may include: a pre-processing step of decreasing a contact angle of a side of a first sheet, which has a plate shape and does not pass excretion, to a water by performing corona treatment on the first sheet; and a sensing pattern forming step of forming a sensing pattern that can transmit and receive a sensing signal indicating the excretion by printing or coating a conductive ink containing water on a side of the first sheet increased in hydrophilicity through the corona treatment.

## Description

### Technical Field

The present invention relates to a method for manufacturing an absorption product that holds excretion discharged from the body of a user, and a pattern roll.

### Background Art

A diaper is an absorptive product that the elderly or people with disability wear and there are a disposable diaper, training pants, pants for urinary incontinence, etc. These absorptive products are all used separately in accordance with the lifestyle or the nursing level of the wearers.

For example, a disposable diaper holds and absorbs excretion without leaking the excretion for people who has difficulty in excreting by themselves such as the elderly or people with disability, so such a disposable diaper is used as an assistant product for excretion etc., or a sanitary product for nursing care.

Urine may be an important data in finding out the health condition of a user. Accordingly, a means that monitors and manages urine of a user and a method of manufacturing the means are required.

### Disclosure

### Technical Problem

The present invention provides a method for manufacturing an absorption product having a sensor function that senses excretion of a user, and a pattern roll having a sensing pattern that senses excretion.

### Technical Solution

A method for manufacturing an absorption product of the present invention may include: an unrolling step of unrolling a first sheet having a plate shape and at least partially not passing excretion from a source roll on which the first sheet is rolled; a sensing pattern forming step of forming a sensing pattern, which can transmit and receives a sensing signal indicating excretion, on the first sheet unrolled from the source roll; and a rolling step of forming a sensing pattern roll by rolling the first sheet having the sensing pattern thereon.

A pattern roll of the present invention includes: a first sheet extending in a first direction; a sensing pattern printed or coated on the first sheet, and continuously extending in the first direction; and a sensing pattern roll on which the first sheet with the sensing pattern printed or coated thereon is rolled about a virtual axis when the virtual axis is defined to extend in a second direction perpendicular to the first direction, in which a rear end of the sensing pattern may coincide with a rear end of the first sheet exposed on an outer surface of the sensing pattern roll.

### Advantageous Effects

According to the method of manufacturing an absorption product of the present invention, a sensing pattern through which sense a sensing signal of a terminal unit can be transmitted may be formed on an absorption product that holds excretion.

A first sheet made of a material at least partially not passing excretion may be disposed on the absorption product to prevent leakage of the excretion.

The first sheet may have difficult to absorb a conductive ink for forming a sensing pattern due to a smooth surface. If the conductive ink is not normally absorbed, it is difficult to expect sensing of excretion from a sensing signal transmitted through the sensing pattern.

Accordingly, it is required to improve the degree of absorption of the conductive ink in the first sheet in order to accurately form a setting gap, a setting width, and a sensing pattern satisfying the setting width.

According to the method of manufacturing an absorption product of the present invention, the degree of absorption of a conductive ink containing water can be improved by applying corona treatment to a side of the first sheet on which the conductive ink is printed.

Accordingly, a plurality of sensing patterns having a setting gap and a setting width can be accurately formed on the first sheet. The plurality of sensing patterns formed to fit to the setting gap and the setting width can function as a sensor that senses excretion wetting the first sheet with high precision.

The method of manufacturing an absorption product of the present invention can unwind a source roll on which a first sheet is rolled at a first position and roll the first sheet on a sensing pattern roll at a second position, and can perform corona treatment and sensing pattern treatment on the first sheet continuing between the source roll and the sensing pattern roll. Therefore, according to the present invention, a roll-to-roll type in which the source roll is input and the sensing pattern roll is output is applied, convenience of inputting and outputting the first sheet in a roll state can be provided.

The method of manufacturing an absorption product of the present invention can form a plurality of sensing patterns to fit to the gap of terminals of a terminal unit that senses excretion, and can form a marking line to fit to a guide of the terminal unit. Accordingly, the sensing pattern of an absorption product using the first sheet manufactured in accordance with the present invention can be easily aligned to the terminals of the terminal unit by the marking line.

The method of manufacturing an absorption product of the present invention can simultaneously produce a plurality of first sheets for absorption products at one time by forming and slitting a plurality of sensing patterns on the first sheet that is wider than an absorption product.

A pattern roll of the present invention may be a first sheet on which a sensing pattern is printed or coated is rolled a plurality of times. Accordingly, by producing an absorption product such as a diaper using the first sheet rolled on the pattern roll, an absorption product having a sensing function that can sense excretion can be manufactured.

### Description of Drawings

FIG. 1 is a flowchart showing a method for manufacturing an absorption product of the present invention.
FIG. 2 is a schematic view of an apparatus for manufacturing an absorption produce of the present invention seen from a side.
FIG. 3 is a schematic view of the apparatus for manufacturing an absorption produce of the present invention seen from above.
FIG. 4 is a schematic view showing an absorption product manufactured by the apparatus for manufacturing an absorption produce of the present invention.
FIG. 5 illustrates schematic views showing a cross-section of a sheet part.
FIG. 6 is a schematic view showing an absorption product of the present invention in which a sensing pattern and a marking line are formed together.
FIG. 7 is a schematic view showing a pattern roll of the present invention.
FIG. 8 is a schematic view showing a state in which a first sheet is wound around a sensing pattern roll.
FIG. 9 is a schematic view showing a cutting process of a first sheet and a second sheet bonded to each other.

### Mode for Invention

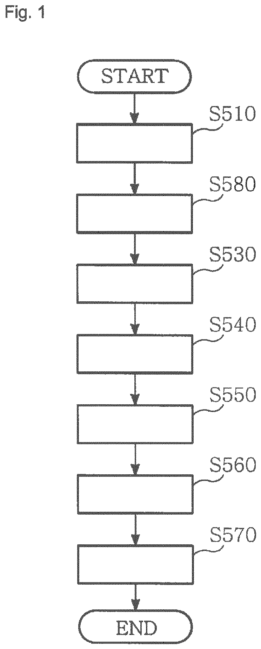

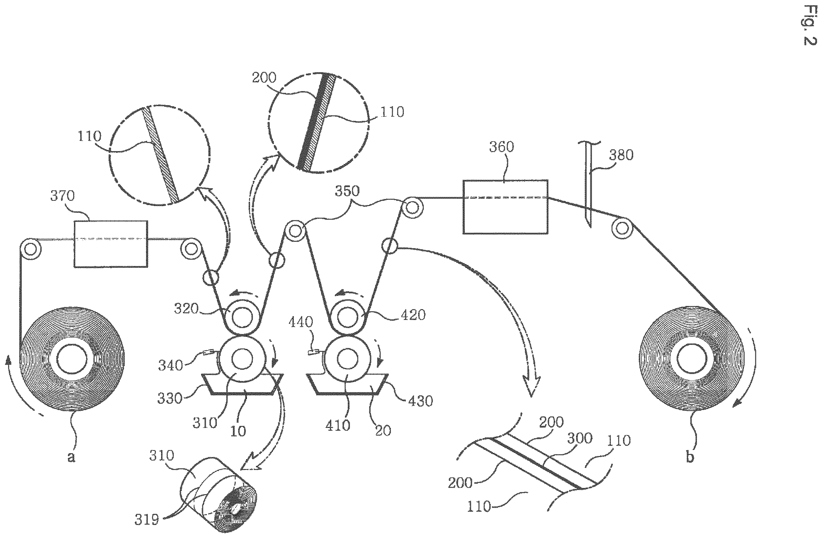

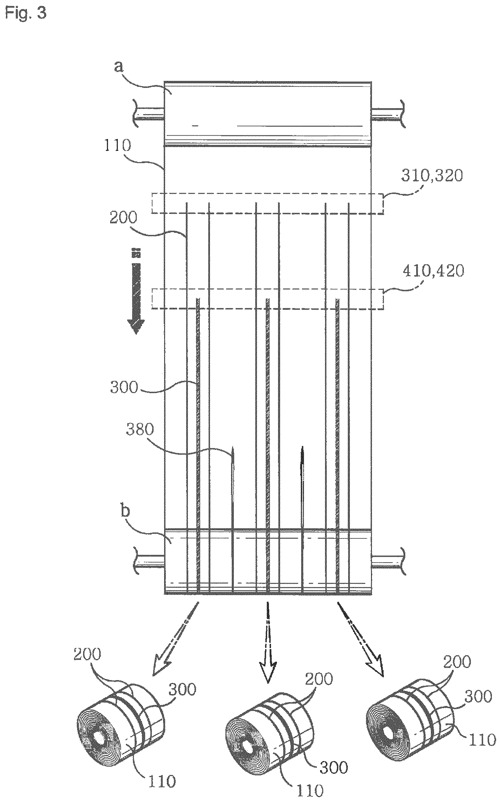

FIG. 1 is a flowchart showing a method for manufacturing an absorption product of the present invention. FIG. 2 is a schematic view of an apparatus for manufacturing an absorption product of the present invention seen from a side. FIG. 3 is a schematic view of the apparatus for manufacturing an absorption produce of the present invention seen from above.

A method for manufacturing an absorption product shown in FIG. 1 may include: an unrolling step of providing a first sheet 110 (S510); a pre-processing step of applying corona treatment to the first sheet 110 (S580); a sensing pattern forming step of forming a sensing pattern 200 on the first sheet 110 (S530); a marking line forming step of forming a marking line 300 on the first sheet 110 (S540); a drying step of drying the sensing pattern 200 or the marking line 300 (S550); a slitting step of slitting the first sheet 110 having a large width into a plurality of first sheets 110 having a small width (S560); and a rolling step of forming a sensing pattern roll 'b' by rolling the first sheet 110 having the sensing pattern 200 thereon.

The unrolling step (S510) corresponds to a step of preparing and providing the first sheet 110.

The first sheet 110 is formed in a plate shape and may include vinyl, film, etc. that at least partially blocks excretion. For example, the first sheet 110 may include polyethylene.

The first sheet 110 may be disposed in an excretion area that holds excretion of a user in the absorption product.

The excretion of a user may include excrement, urine, sweat, menstrual blood, tears, saliva, etc.

In order to hold the excretions, the absorption product may include the sheet part 100 such as a diaper or a pad that holds excrement and urine, a patch that is attached to a specific position of the user's body and holds sweat, a hygienic band that holds menstrual blood, an eye patch that holds tears, a mask that holds saliva, etc..

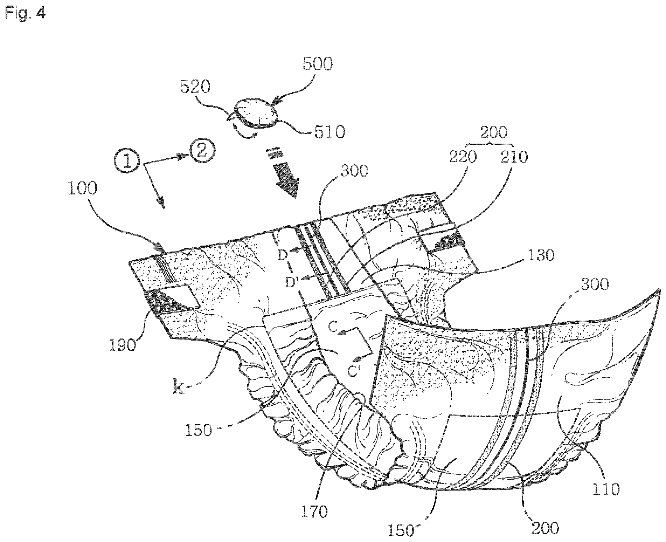

The sheet part 110 shown in FIG. 4 may include a diaper that is put between the legs of a user to hold excrement and urine. The parts shown in the figures may be applied in the same way to other kinds of sheet parts 100 such as a hygienic band.

The first sheet 110 may be fabricated by forming polyethylene powder.

A source roll 'a' of the first sheet 110 without the sensing pattern 200 formed thereon may be prepared in the unrolling step (S510) to improve convenience in handling.

The unrolling step (S510) may include a step of unrolling the first sheet 110 from the source roll 'a' on which the first sheet 110 having a plate shape and at least partially blocks excretion.

The first sheet 110 that is manufactured in the method for manufacturing an absorption product of the present invention may be a part for forming a sheet part 110 on which a terminal unit 500 having terminals 531, 532, 533, and 534 that apply sensing signals.

The sensing pattern 200 that transmits/receives a sensing signal to/from the terminal unit 500 in physical contact with the terminals may be formed on the sheet part 100. The sensing pattern may be referred to as a patch sensor, a patch film, a sensor patch, etc.

The sensing pattern 200 that can transmit/receive a sensing signal may be made of a conductive ink 10. A plurality of terminals spaced apart from each other may be formed at the terminal unit 500 to sense excretion using sensing signals. A positive (+) sensing pattern 210 with which some terminals are in contact and a negative (-) sensing pattern 220 with which other terminals are in contact may be formed on the sheet part 100.

The positive sensing pattern 210 and the negative sensing pattern 220 are spaced apart from each other, so a sensing signal that is applied to the positive sensing pattern 210 in an initial state without excretion cannot be transmitted to the negative sensing pattern 220. If excretion is distributed on the positive sensing pattern 210 and the negative sensing pattern 220, a sensing signal of the positive sensing pattern 210 may be transmitted to the negative sensing pattern 220 through the excretion. The terminal unit 500 can sense excretion or determine the amount of excretion by analyzing a sensing signal transmitted to the negative sensing pattern 220.

The thickness, width, etc. of the sensing patterns 220 have to satisfy setting values for normal operation of the terminal unit 500. However, it may be difficult to print the conductive ink 10 with the thickness and width that satisfy the setting values on the first sheet 110 having a smooth surface.

The pre-precession step (S580) may be performed before the sensing pattern-forming step (S530) that forms the sensing pattern 200 in order to form the sensing pattern 200 having a thickness and a width that satisfy setting values.

The pre-processing step (S580) may perform corona treatment on the first sheet 110 unrolled from the source roll 'a'.

For example, the pre-processing step (S580) can reduce a contact angle with water of a side of the first sheet 110 by applying corona treatment on the first sheet 110 having a plate shape and blocks excretion.

The corona treatment corresponds to a discharge treatment and is a method of reforming a surface by producing corona salts using a discharge treatment unit 370 and passing the first sheet between the corona salts. The surface of the first sheet 110 is oxidized by the corona treatment, so the contact angle with water that indicates the degree of hydrophilicity decreases. As a result, printability of the first sheet 110 of polyethylene is greatly improved, so a precise sensing pattern 200 having a thickness and a width of setting values can be formed using the conductive ink 10.

The discharge treatment unit 370 that applies corona treatment on the first sheet 110 unrolled from the source roll 'a' may be disposed between sensing pattern unit 310, 320 that forms the sensing pattern 200 and the source roll 'a'.

The sensing pattern unit can form the sensing pattern 200 by printing or coating a conductive ink on the first sheet 110 increased in hydrophilicity through the pre-processing step (S580).

The sensing patter forming step (S530) can form the sensing pattern 200 that can transmit/receive a sensing signal indicating excretion on the first sheet 110 unrolled from the source roll 'a'.

For example, the sensing pattern forming step (S530) can form the sensing pattern 20 that can transmit/receive a sensing signal indicating excretion by printing or coating the conductive ink 10 containing water on a side of the first sheet 110 increased in hydrophilicity through corona treatment.

The conductive ink 10 may include at least one (conductive substance such as a carbon-based substance, a polymer-based substance, a metal oxide, and PEDOT) of substances including a metal oxide such as carbon that conducts electricity and a conductive polymer, a solvent that thins down a conductive substance such as a carbon-based substance, a polymer-based substance, a metal oxide, and PEDOT, and water and acetyl corresponding to a dissolvent of a conductive polymer.

PEDOT is poly(3,4-ethylenedioxythiophene).

The conductive polymer may include polyacetylene, polypyrrole, polythiophene, polyaniline, etc.

The sensing pattern forming step (S530) can print or coat a plurality of sensing patterns 200, which passes through an excretion area 'k' of the first sheet 110 that holds excretion, to be spaced apart from each other on the first sheet 110 so that the equivalent resistance of the sensing pattern 200 is changed by excretion. In this case, the sensing patterns 200 may be continuously printed or coated on the first sheet 110 in the direction in which the first sheet 110 is unrolled from the source roll 'a'.

A sensing pattern unit that forms the sensing pattern 200 on the first sheet 110 may be disposed between the source roll 'a' and the sensing pattern roll 'b'.

The first sheet 110 can continuously pass through the sensing pattern unit while being continuously unrolled from the source roll 'a'.

The sensing pattern 200 can be continuously formed by the sensing pattern unit on the first sheet 110 continuously passing through the sensing pattern unit.

The first sheet 110 that has continuously passed through the sensing pattern unit can be continuously rolled on the sensing pattern roll 'b'.

The sensing pattern roll 'b' may be the first sheet having the sensing pattern and wound on a cylindrical core. Depending on cases, the sensing pattern roll 'b' may be formed without a core.

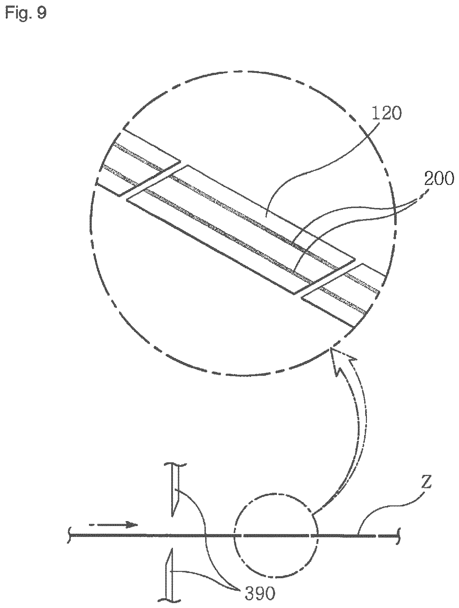

As in FIG. 9, the first sheet 110 rolled on the sensing pattern roll 'b' can be cut to fit to the length of an absorption product by a cutting unit 390 that performs a post-process that manufactures an absorption product. It is realistically difficult to roll the first sheet 110 of an infinite length on the sensing pattern roll 'b'. The first sheet 110 can be cut in the unit of a setting length, and even in this case, the first sheet can be cut by the cutting unit. When the first sheet rolled in the source roll 'a' has a limited length, a sensing pattern roll 'b' of the first sheet having the limited length can be formed even without using a specific cutting unit.

In this case, in order that a plurality of sensing patterns respectively comes in contact with a plurality of terminals formed at the terminal unit that generates a sensing signal regardless of a cut position of the first sheet 110, the sensing pattern forming step (S530) can form a plurality of sensing patterns 200 with same intervals in parallel.

The sensing pattern forming step (S530) can continuously form the sensing pattern 200 in the longitudinal direction of the first sheet 110 such that the rear end of the cut first sheet 110 and the rear end of the sensing pattern 200 formed on the cut first sheet 110 coincide with each other regardless of the cut position of the first sheet 110.

When the sensing pattern 200 includes a first sensing pattern and a second sensing pattern that are spaced apart from each other, the rear end of the first sensing pattern and the rear end of the second sensing pattern may be both positioned at the rear end of the cut first sheet 110. For example, the first sensing pattern may include a positive sensing pattern 210 of FIG. 4 and the second sensing pattern may include a negative sensing pattern 220 of FIG. 4.

The sensing pattern forming step (S530) may form a plurality of sensing patterns 200 spaced apart from each other to fit to the gaps of the plurality of terminals formed at the terminal unit 500 that generates a sensing signal indicating excretion.

For example, the second pattern 200 may be formed on the first sheet 110 through gravure printing.

The sensing pattern forming step (S530) may be performed by the sensing pattern unit disposed between the source roll 'a' and the sensing pattern roll 'b'.

The sensing pattern unit may include a first cylindrical plate gravure rotary press 310 that is disposed on a path of the first sheet 110 moving from the source roll 'a' to the sensing pattern roll 'b' and rotates in pace with movement of the first sheet 110.

The sensing pattern forming step (S530) can continuously print or coat the sensing pattern 200 on the first sheet 110 that is input to a first flat plate gravure rotary press 320 and the first cylindrical plate gravure rotary press 310, using the first flat plate gravure rotary press 320 and the first cylindrical plate gravure rotary press 310 that are disposed on the movement path of the first sheet 110.

There may be provided a first ink container 330 in which the conductive ink 10 in a liquid or powder state is accommodated, the first cylindrical plate gravure rotary press 310 that rotates with the outer circumferential surface passing through the first ink container 330 and has an engraved shape of the sensing pattern 200 formed on the outer circumferential surface, and the first flat plate gravure rotary press 320 disposed opposite to the first cylindrical plate gravure rotary press 310. Trenches 319 corresponding to a plurality of engraved ribs spaced with the same intervals of the plurality of terminals may be formed on the outer circumferential surface of the first cylindrical plate gravure rotary press 310.

In this case, the plurality of trenches 319 may extend in parallel with each other.

The trenches 319 may be formed in closed curve shapes with the start and the end connected on the outer circumferential surface of the first cylindrical plate gravure rotary press 310 regardless of whether the first sheet 110 is inclined in the longitudinal direction. The plurality of trenches 319 may be formed in parallel with same intervals in the width direction of the first sheet 110.

Each of the trenches 319 may be continuously formed without disconnection along the outer circumference of the first cylindrical plate gravure rotary press 310. The conductive ink existing in the trenches can be printed or coated on a side of the first sheet 110.

A plurality of sensing patterns 200 that is parallel with each other can be continuously formed without disconnection by the trenches 319 on the first sheet 110 that has passed by the first cylindrical plate gravure rotary press 310 in close contact with the first cylindrical plate gravure rotary press 310 that is rotating.

For example, the conductive ink 10 is filled in the plurality of trenches 319 that has passed through the ink container 330 and the conductive ink 10 can be printed on the opposite first sheet 110. The plurality of sensing patterns 200 printed on the first sheet 110 by the engraved ribs can be spaced with the same intervals as the gaps of the plurality of terminals.

A first doctor blade 340 that adjusts the thickness of the conductive ink 10 sticking to the outer circumferential surface and pressing rollers 350 keeping the first sheet 110 tight may be additionally provide at positions facing the first cylindrical plate gravure rotary press 310.

The first sheet 110 unrolled from the source roll 'a' is input between the rotary presses and can be printed or coated with the conductive ink 10 between the rotary presses. In this case, the conductive ink 10 printed or coated on a side of the first sheet 110 can be naturally dried or forcibly dried by a drying unit 360 while forming the sensing pattern 200 that can transmit/receive a sensing signal. The first sheet 110 with the conductive ink 10 dried becomes a first sheet 110 having the sensing pattern 200, and the first sheet 110 having the sensing pattern 200 can be rolled again.

When the source roll 'a' of the first sheet 110 is disposed at a first position and the sensing pattern roll 'b' is disposed at a second position, the first sheet 110 unrolled from the source roll 'a' continues to the second position and can be rolled on the sensing roll pattern 'b' at the second position.

In this case, the pre-processing step (S580), the sensing pattern forming step (S530), the marking line forming step (S540), the drying step (S550), and the slitting step (S560) are performed between the first position and the second position and may be performed on the first sheet 110 continues from the first position to the second position.

According to the embodiment, the first sheet 110 can be input in a source roll state and the first sheet 110 that has undergone various steps can be output in a sensing pattern roll state.

The marking line forming step (S540) can form a marking line of which the color changed when the first sheet 110 is stained with excretion. The marking line forming step (S540) can be performed by a making unit 410, 420 disposed between the sensing pattern unit, which performs the sensing pattern forming step (S530), and the source roll 'a' or disposed between the sensing pattern unit and the sensing pattern roll 'b'.

The marking line forming step (S540), which prints or coats a water-discoloring ink 20 containing water on a side of the first sheet 110 with hydrophilicity increased through corona treatment, can form a marking line 300 that discolors when it is stained with excretion.

The marking line 300 may be formed to discolor when it is stained with excretion.

The marking line 300 may include a water-discoloring ink 20. The water-discoloring ink 20 may contain a CoCL₂ paint that discolors depending on the degree of absorption of water. Obviously, all kinds of paints that discolor depending on the degree of absorption of water may be used other than such a paint.

The marking line forming step (S540) can form the marking line 300 on the first sheet 110 through gravure printing.

The making line forming step (S540) can continuously print or coat the marking line 300 on the first sheet 110 that is input to a second flat plate gravure rotary press 420 and a second cylindrical plate gravure rotary press 410, using the second flat plate gravure rotary press 420 and the second cylindrical gravure rotary press 410 that are disposed on the movement path of the first sheet 110.

There may be provided a second ink container 430 in which the water-discoloring ink 20 in a liquid or powder state is accommodated, the second cylindrical plate gravure rotary press 410 that rotates with the outer circumferential surface passing through the second ink container 430 and has an engraved shape of the sensing pattern 200 formed on the outer circumferential surface, and the second flat plate gravure rotary press 420 disposed opposite to the second cylindrical plate gravure rotary press 410. A second doctor blade 440 that adjusts the thickness of the water-discoloring ink 20 sticking to the outer circumferential surface and pressing rollers 350 keeping the first sheet 110 tight may be additionally provided at positions facing the first cylindrical plate gravure rotary pressure 410.

The marking unit such as the second flat plate gravure rotary press 420 that performs the marking line forming step (S540) may be disposed ahead of or behind the sensing pattern unit such as the first flat plate gravure rotary press 420 that performs the sensing pattern forming step (S530). Accordingly, the marking line forming step (S540) may be performed before or after the sensing pattern forming step (S530). In the drawings, the marking line forming step (S540) is performed after the sensing pattern forming step (S530).

The marking line forming step (S540) can form the making line 300 spaced apart from the sensing pattern 200 to fit to the gap between a guide 521 of the terminal unit 500, which guides alignment between the terminals of the terminal unit 500 and the sensing pattern 200 of the first sheet 1, and the terminals.

The marking line forming step may be attaching a litmus paper for testing acidity or alkalinity of excretion to a side of the first sheet or printing or coating a litmus coloring matter to a side of the first sheet.

The marking line forming step may be combined with the sensing pattern forming step as one step. For example, the sensing pattern forming step prints or coats a conductive ink to a side of the first sheet, in which a litmus coloring matter for testing acidity or alkalinity of excretion may be contained in the conductive ink. In this case, the sensing pattern formed through the sensing pattern forming step may have all of an excretion sensing function that uses a sensing signal, a marking line function for excretion, and an excretion PH (pH value) determination function.

The drying step (S550) can forcibly dry the conductive ink 10 formed on a side of the first sheet 110 using the drying unit 360 after the sensing pattern forming step.

The drying unit 365 can dry the conductive ink 10 or the water-discoloring ink 20 sticking to the sensing patter 200 using a heater, hot air, etc. The drying unit 360 may be disposed between the sensing pattern unit and the sensing pattern roll 'b'. The drying unit 360 can dry the sensing pattern 200 formed on the first sheet 110 before the first sheet 110 is rolled on the sensing pattern roll 'b'.

The drying step (S550) is performed on the first sheet 110 after both of the sensing pattern forming step (S530) and the marking line forming step (S540) are finished, and additionally, may be provided between the sensing pattern forming step (S530) and the marking line forming step (S540).

The pre-processing step (S580), the sensing pattern forming step (S530), and the marking line forming step (S540) may be performed on a first sheet 110 having a width over an integer time of the width of an absorption product that holds excretion.

The sensing pattern forming step (S530) or the marking line forming step (S540) can form the sensing pattern 200 or the marking line 300 at every area or position corresponding to each absorption product in the width direction of the first sheet 110.

The slitting step (S560) can form a plurality of first sheets 110 having a width for manufacturing absorption products by slitting one first sheet 110, which has a width over an integer time of the width of the absorption products, using a slitting unit after the sensing pattern forming step and the marking line forming step (S540).

When the first sheet 110 that has undergone the sensing pattern forming step (S530) or the marking line forming step (S540) is rolled on the sensing pattern roll 'b', the slitting step (S560) may be performed using a slitting unit 380 such as a cutter disposed between the sensing pattern unit performing the sensing pattern forming step (S530) and the sensing pattern roll 'b' or may be performed using a slitting unit 380 disposed between the marking unit performing the marking line forming step (S540) and the sensing pattern roll 'b'.

In some cases, the slitting step (S560) may be performed by cutting the sensing pattern roll 'b'.

The slitting unit 380 can form a plurality of first sheets 110 having a second width smaller than a first width by cutting the first sheet 110, which has the first width and moves toward the sensing pattern roll from the sensing patter unit, between the sensing pattern unit and the sensing pattern roll. Alternatively, the slitting unit 380 can form a plurality of first sheets 110 having a second width smaller than a first width by cutting the first sheet 110, which has the first width and moves toward the sensing pattern roll from the marking unit, between the marking unit and the sensing pattern roll.

The first plurality of first sheets 110 cut in the longitudinal direction by the slitting unit 380 may be rolled on the sensing pattern roll 'b' in the rolling step (S570).

The first sheets 110 having the second width by the slitting unit 380 can be sent to the next step that forms absorption product in the state of the sensing pattern roll 'b' having the second width.

When the first sheet 110 that has undergone the sensing pattern forming step (S530) is rolled on the sensing pattern roll, a sub-roll of a second sheet 120 made of a material that passes excretion may be provided through the next step of manufacturing absorption products.

A sensing pattern roll and a sub-roll may be installed to put a second sheet 120 unrolled from the sub-roll onto the side on which the sensing pattern 200 is printed or coated in the first sheet 110 unrolled from the sensing pattern roll.

The first sheet 110 and the second sheet 120 can be moved together to a bonding roller so that the second sheet 120 is put on the side of the first sheet 110 on which the sensing pattern 200 is printed or coated.

The second sheet 120 can be bonded to the side of the first sheet 110 on which the sensing pattern 200 is printed or coated by the bonding roller. The first sheet and the second sheet can be combined in the same size. The second sheet can be put on the first sheet with the sensing pattern therebetween.

The terminals of the terminal unit can come in close contact with the second sheet 120 and can come in contact with the sensing pattern on the first sheet through the second sheet.

When the first sheet includes a material that passes some of excretion, the terminals of the terminal unit can in close contact with the sensing pattern on the first sheet in directly close contact with the first sheet 110.

The first sheet 110 and the second sheet 120 bonded to each other and discharged from the bonding roller can be cut by the cutting unit 390 to fit to the length of an absorption product that holds excretion. When the bonded sheet z of the first sheet and the second sheet combined with each other is cut, the rear end of the sensing pattern may coincide with the rear end of the first sheet and the rear end of the second sheet.

The first sheet 110 and the second sheet 120 cut to fit to the length of an absorption product can be tailored to fit to the shape of the absorption product.

An absorber 150 that absorbs excretion may be stacked on the center of the second sheet 120 tailored to fit to an absorption product and bonded to the first sheet 110.

The absorber 150 stacked on the center of the second sheet 120 is covered with a third sheet 130 made of a material that passes excretion and then the edge of the third sheet 130 is bonded to the edge of the second sheet 120, whereby the sheet part 100 corresponding to an absorption product having the sensing pattern 200 can be completed.

FIG. 4 is a schematic view showing an absorption product manufactured by the apparatus for manufacturing an absorption produce of the present invention.

FIG. 4 shows a diaper that is put between the legs of a user to hold excrement and urine as an example of the sheet part 100 according to an absorption product.

A pair of tape fasteners 190 may be disposed at an edge of the diaper. The tapes of the taper fasteners 190 can adhere to the other edge of the diaper.

A pair of cuffs 170 being erect upward to prevent leakage may be disposed at both sides of the center portion of the diaper.

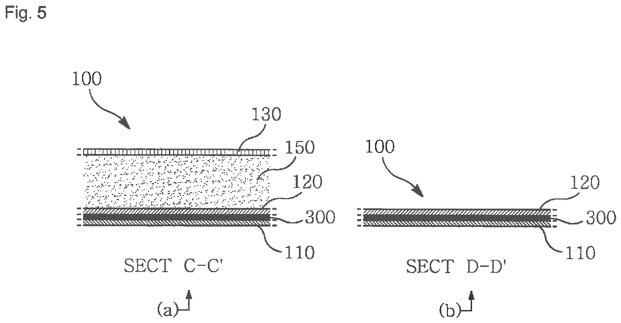

FIG. 5 illustrates schematic views showing a cross-section of the sheet part 100.

The sheet part 100 may include a first sheet 110 made of a material not passing excretion and a second sheet 120 stacked on a side of the first sheet 110 that is supposed to face a user and passes excretion. Further, a sensing pattern 200 printed on a side of the first sheet 110 that faces the second sheet 120 and a marking line 300 printed on the side of the first sheet 110 that faces the second sheet 120 may be provided on the sheet part 110.

The first sheet 110 may include a transparent material so that the making line 300 formed on the side of the first sheet 110 is visually seen through the other side of the first sheet 110.

When the marking line 300 is stained with water of excretion, the color of the marking line 300 can change. A user can easily find out whether there is excretion by observing a color change of the marking line 300 through the first sheet 110 made of a transparent material.

The first sheet 110 may be partially changed in shape in consideration of the wearing position of the sheet part 100. The first sheet 110 may be formed fundamentally in a plate shape wider than the excretion organ in order to securely hold a large amount of excretion without leakage.

An absorber 150 may be added to increase the ability to absorb excretion.

The absorber 150 may be stacked on a side of the second sheet 120 that is supposed to face a user. The absorber 150 may include materials such as cotton, SAP (Super Absorbent Polymer), a tissue, etc.

The absorber 150 made of cotton, etc. easily slides on the first sheet 110 made of vinyl. In this case, the cotton particles, SAP particles, and tissue particles constituting the absorber 150 are changed in position without being fixed with respect to the first sheet 110 due to the sliding, so an abnormal phenomenon that the absorber 150 is biased to any one side with the other side empty in the excretion area may occur.

The second sheet 120 that is easily bonded to the first sheet 110 made of vinyl may be used to prevent a position change of the particles of the absorber 150. For example, the second sheet 120 that is brought in contact with cotton may include fabric passing excretion and limiting a position change of the cotton.

When the absorber 150 is provided, a third sheet 130 may be added to prevent a phenomenon that the absorber 150 is separated from the second sheet 120.

The third sheet 130 is stacked on a side of the absorber 150 that is supposed to face a user, and may include a material that passes excretion. The center portion of the third sheet 130 covers the absorber 150 and the edges of the third sheet 130 can be bonded directly to the first sheet 110 or the second sheet 120.

The center portion of the sheet part 100 including the absorber 150, as shown in FIG. 5(a) showing the cross-section of line C-C' of FIG. 4, may formed in a structure in which the first sheet 110, the marking line 300, the second sheet 120, the absorber 150, and the third sheet 130 are sequentially stacked. The sensing pattern 200 may be positioned in the same layer as the marking line 300 and may be positioned at different position from the marking line 300 in the plane.

The edges of the sheet part 100, as shown in FIG. 5(b) showing the cross-section of line D-D' of FIG. 4, may be formed in a structure in which the first sheet 110, the marking line 300, and the second sheet 120 are sequentially stacked. The sheet pattern 200 may be disposed in the same layer as the marking line in the cross-section. The third sheet 130 may be additionally stacked on the second sheet 120 at the edges of the sheet part 100.

The third sheet 130 can function as a cover that prevents separation of the absorber 150 from the first sheet 110 or the second sheet 120. Further, the third sheet 130 can prevent a phenomenon that the cotton particles, the SAP particles, and the tissue particles constituting the absorber 140 stick to the user's body or excretion absorbed in the absorber 150 sticks back to the user's body.

The absorber 150 and the sensing pattern 200 may be formed in the excretion area k. The first sheet 110, the second sheet 120, the absorber 150, and the third sheet 130 may be formed to have areas wider than the excretion area. However, the absorber 150 may be formed to fit to the size of the excretion area to reduce the cost. In other words, the absorber 150 may be formed to have an area smaller than the first sheet 110, the second sheet 120, and the third sheet 130. If the area of the absorber 150 is small, the edges of the third sheet 130 can be easily bonded to the edges of the second sheet 120.

According to this configuration described above, the sheet part 100 in which the first sheet 110, the second sheet 120, the absorber 150, and the third sheet 130 are sequentially stacked in accordance with the direction facing the user's body from the outside can be provided.

Since the second sheet 120, the absorber 150, and the third sheet 130 include the material that passes excretion, excretion discharged in the excretion area from a user may exist from the side of the first sheet 110 that is supposed to face the user to the third sheet 130.

The sensing pattern 200 and the marking line 300 are provided to sense excretion discharged to the excretion area, so they may be disposed within a section from the first sheet 110 to the third sheet 130 where excretion may exist.

The sensing pattern 200 and the marking line 300 can be printed or coated on a side of the first sheet 110 that faces the second sheet 120.

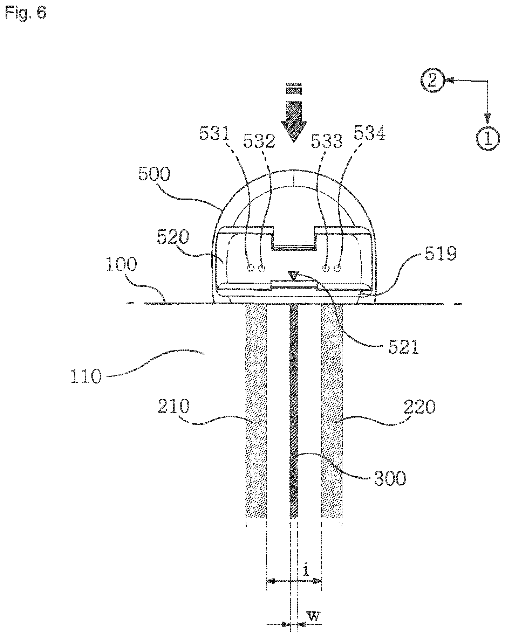

FIG. 6 is a schematic view showing an absorption product of the present invention in which the sensing pattern 200 and the marking line 300 are formed together.

Two or more sensing patterns 200 may be provided for one sheet part 100, in which some sensing patterns of a plurality of sensing patterns 200 may form a positive (+) sensing pattern 210 to which a sensing signal of the terminal unit 500 is applied, and the other sensing patterns of the plurality of sensing patterns 200 may form a negative (-) sensing pattern 220 from which the sensing signal is sent back to the terminal unit 500. The positive sensing pattern 210 and the negative sensing pattern 220 may extend in the longitudinal direction of the first sheet 110 and may be formed to be spaced apart from each other in the width direction of the first sheet 110.

In this case, the marking line 300 may be disposed between the positive sensing pattern 210 and the negative sensing pattern 220. The marking line 300 formed between the two sensing patterns 200 is disposed at the center in the width direction of the first sheet 110, so it has an advantage of high visibility.

The terminals of the terminal unit 500 can be physically in contact with the positive sensing pattern 210 and the negative sensing pattern 220, respectively. The terminal unit 500 can apply a sensing signal to the terminal being in contact with to the positive sensing pattern 210.

The sensing signal applied to the positive sensing pattern 210 is not transmitted to the negative sensing pattern 220 spaced apart from the positive sensing pattern 210.

When the positive sensing pattern 210 and the negative sensing pattern 220 are stained with excretion, the positive sensing pattern 210 and the negative sensing pattern 220 come in to a conductive state due to the excretion that conducts electricity. Accordingly, the sensing signal applied to the positive sensing pattern 210 is transmitted to the negative sensing pattern 220 through the excretion, and the sensing signal transmitted to the negative sensing pattern 220 can be provided to the terminal unit 500 through the terminal being physically in contact with the negative sensing pattern 220.

The terminal unit 500 can find out whether there is excretion, the amount of excretion, etc. by analyzing the sensing signal acquired from the terminal being in contact with the negative sensing pattern 220.

However, a sensing signal may be almost not transmitted from the positive sensing pattern 210 and the negative sensing pattern 220 due to low electricity conductivity of excretion even if the excretion exists across the positive sensing pattern 210 and the negative sensing pattern 220. The larger the distance i between the positive sensing pattern 210 and the negative sensing pattern 220, the more severe the phenomenon may become.

The marking line 300 may be used so that a sensing signal applied to the positive sensing pattern 210 can be securely transmitted to the negative sensing pattern 220 through excretion.

For example, the marking line 300 disposed between the positive sensing pattern 210 and the negative sensing pattern 220 may be disposed at a position spaced apart from the positive sensing pattern 210 and the negative sensing pattern 220. In this case, the marking line 300 may include a conductive material that transmits a sensing signal.

If the distance that a sensing signal has to travel without the marking line 300 is I, when the marking line 300 made of a conductive material and having a width w is disposed between the sensing patterns 200, the distance that the sensing signal has to travel can be reduced to the value obtained by subtracting w from i. In other words, the resistance value of excretion existing across the sensing patterns 200 can be reduced by the marking line 300, and a sensing signal can be easily transmitted to the negative sensing pattern 220 due to the reduced resistance value.

When the marking line 300 made of a conductive material is disposed between the sensing patterns 200, excretion can be securely sensed by the marking line 300 and the sensing patterns 200 even if the amount of the excretion is very small such that a sensing signal is difficult to be transmitted.

On the other hand, the marking line 300 can guide alignment between the sheet part 100 and the terminal unit 500.

The terminal unit 500 may have terminals being physically in contact with the sensing patterns 200 and a guide 521 that is formed at a position spaced from the terminals in the width direction of the first sheet 110 and guides the terminals to be aligned to the sensing patterns 200.

In this case, the marking line 300 may be formed at a position that is aligned to the guide when the terminals are aligned to the sensing patterns 200.

According to the embodiment, when a user aligns the guide 521 to the marking line 300, the terminals of the terminal unit 500 can be naturally aligned to the sensing patterns 200.

In order to guide alignment of the guide 521, the marking line 300 may have a color different from those of the first sheet 110 and the second sheet 120 in the initial state. According to the marking line 300 of the embodiment, it is not required to form the sensing patterns 200 to have a color for alignment between the terminals and the sensing patterns 200. Accordingly, if the marking line 300 is added, the sensing patterns 200 may have a white color or may have no color.

The terminal unit 500 may have a first attachment/detachment portion 510 and a second attachment/detachment portion 580 that are coupled to each other with the sheet part 100 therebetween in order to be attached/detached to/from the sheet part 100.

A seat 519 in which the sheet part 100 is inserted may be disposed at the first attachment/detachment portion 510.

Terminals formed at the seat 519 of the first attachment/detachment portion 510 of the terminal unit 500, for example, a first terminal 531, a second terminal 532, a third terminal 533, and a fourth terminal 534 may be hidden by the second attachment/detachment portion 580 covering the terminals with the sheet part 100 therebetween. The first terminal 531 or the second terminal 532 can apply a sensing signal to the positive sensing pattern 210. The third terminal 533 or the fourth terminal 534 can apply a sensing signal to the negative sensing pattern 220.

Accordingly, a user has to maintain the alignment state between the sensing patterns 200 and the terminals by depending only on a sense from the point in timing when the terminals are hidden by the second attachment/detachment portion 580.

The glide 521 may be formed on the outer surface of the second attachment/detachment portion 580 covering the terminals. Accordingly, the guide 521 may be exposed outside with the terminals covered by the second attachment/detachment portion 580. A user can easily align the guide 521 exposed to the outside to the marking line 300.

The distance between the guide 521 and the terminals may be the same as the distance between the marking line 300 and the sensing patterns 200 in a second direction ② corresponding to the width direction of the first sheet 110.

Accordingly, when the position of the terminal unit 500 is adjusted with respect to the sheet part 100 such that the guide 521 is aligned to the marking line 300, the sensing patterns 200 and the terminals can be naturally aligned.

Three or more terminals that are physically in contact with the sensing patterns 200 may be formed at the terminal unit 500. The number of the terminals may be larger by one or more than the number of the sensing patterns 200.

The terminals and the sensing patterns 200 may be formed such that at least one terminal physically comes in contact with each of two or more sensing patterns 200 spaced apart from each other when the terminal unit 500 is mounted on the sheet part 100.

For example, when two sensing patterns 200 spaced a setting gap apart from each other are provided on the sheet part 100, two terminals spaced the same gap as that of the two sensing patterns 200 apart from each other may be provided at the terminal unit 500. In this case, when any one terminal physically comes in contact with a specific sensing pattern 200, the other terminal can be naturally aligned to and physically brought in contact with the other sensing pattern 200.

The terminals and the sensing patterns 200 can be electrically connected by the physical contact between the terminals and the sensing patterns 200.

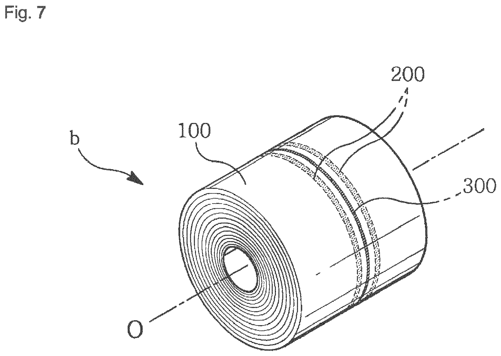

FIG. 7 is a schematic view showing a pattern roll of the present invention.

The pattern roll shown in the figure may include a sensing pattern roll 'b' formed through the sensing step (S570).

For example, the pattern roll may include a first sheet 110 extending in a first direction, a sensing pattern 200 printed or coated on the first sheet 110 and continuously extending in the first direction, and a sensing pattern roll 'b' rolled about a virtual axis 'o' perpendicular to the first direction.

The sensing pattern roll 'b' may be a first sheet having the sensing pattern and wound on a cylindrical core. If necessary, the core may be removed after the sensing pattern roll 'b' is formed.

The first direction may coincide with the longitudinal direction of the first sheet 110 when the first sheet 110 is unfolded.

The length of the first sheet rolled in the sensing pattern roll 'b' may be limited by a limited length of the first sheet supplied from a source roll 'a' or cutting by the cutting unit. As a result, the rear end of the first sheet may be exposed on the outer circumferential surface of the sensing pattern roll 'b'.

In this case, the rear end of the sensing pattern may coincide with the rear end of the first sheet exposed on the outer circumferential surface of the sensing pattern roll 'b'.

According to the sensing pattern roll coinciding with the rear end of the first sheet, a normal absorption product can be manufactured using the rear end of the first sheet without cutting the rear end of the first sheet. Accordingly, a waste of a material is prevented.

Further, alignment with a second sheet, etc. can be easily performed by visually checking the position of the rear end of the sensing pattern coinciding with the rear end of the first sheet.

The sensing pattern 200 formed on the sensing pattern roll 'b' may be provided as a plurality of pieces spaced apart from each other to sense excretion.

In this case, the plurality of sensing patterns may be formed with the same intervals in parallel. The sensing patterns 200 formed on the sensing pattern roll 'b' may be formed to overlap each other when the sensing pattern roll 'b' is seen perpendicularly to the second direction. For example, when two sensing patterns extending in straight line shapes, even if a plurality of sensing patterns are stacked on the sensing pattern roll 'b', only two sensing patterns can be seen when the sensing pattern roll 'b' is seen perpendicularly to the second direction due to overlapping sensing patterns.

A first sheet 110 may be stacked in a plurality of times in the radial direction on the sensing pattern roll 'b' formed by rolling the first sheet 110 having sensing patterns 200 a plurality of times. In this case, the sensing patterns can be stacked between the first sheet and the first sheet stacked on each other. The number of times of stacking the sensing patterns or the number of times of winding sensing patterns on the sensing pattern roll 'b' may be equal to or less than the number of times of stacking the first sheet or the number of times of rolling the first sheet on the sensing pattern roll 'b'. This is because a portion of a first sheet may be rolled on the sensing pattern roll 'b' without a sensing pattern formed in the process of initially rolling the sensing pattern roll 'b' by unrolling a portion of the first sheet of the source roller 'a' in the early stage of the method for manufacturing an absorption product.

When a sensing pattern is formed from the front end of a first sheet that is unrolled from the source roller 'a' and initially connected to the sensing pattern roll 'b', the sensing pattern can be wound on the sensing pattern roll by the number of times of rolling the first sheet on the sensing pattern roll 'b'.

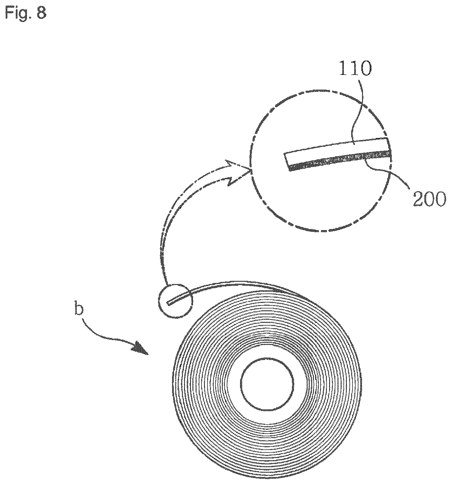

FIG. 8 is a schematic view showing a state in which a first sheet is rolled on a sensing pattern roll.

The sensing pattern roll 'b' may be a roll of the first sheet 110 rolled such that the sensing pattern 200 faces the inside (center) of the sensing pattern roll 'b' and the first sheet 110 faces the outer side (outside) of the sensing pattern roll.

According to the embodiment, since the sensing pattern 200 is covered by the first sheet 110, damage to the sensing pattern 200 due to external shock, etc. can be prevented.

## Claims

1. A method for manufacturing an absorption product, the method comprising:
an unrolling step of unrolling a first sheet having a plate shape and at least partially not passing excretion from a source roll on which the first sheet is rolled;
a sensing pattern forming step of forming a sensing pattern, which can transmit and receive a sensing signal indicating excretion, on the first sheet unrolled from the source roll; and
a rolling step of forming a sensing pattern roll by rolling the first sheet having the sensing pattern thereon.

2. The method of claim 1, wherein a sensing pattern unit forming the sensing pattern on the first sheet is disposed between the source roll and the sensing pattern roll,
the first sheet continuously passes through the sensing pattern unit while being continuously unrolled from the source roll,
the sensing pattern is continuously formed by the sensing pattern unit on the first sheet continuously passing through the sensing patter unit, and
the first sheet that has continuously passed through the sensing pattern unit is continuously rolled on the sensing pattern roll.

3. The method of claim 1, wherein the sensing pattern forming step forms a plurality of sensing patterns, which passes through an excretion area of the first sheet that holds the excretion, to be spaced apart from each other on the first sheet so that equivalent resistance of the sensing pattern is changed by the excretion, and
the sensing pattern forming step forms a plurality of sensing patterns with same intervals in parallel so that the plurality of sensing patterns respectively comes in contact with a plurality of terminals formed at a terminal unit that generates the sensing signal regardless of a cut position of the first sheet.

4. The method of claim 1, wherein the sensing pattern forming step continuously forms the sensing pattern in a longitudinal direction of the first sheet such that a rear end of the cut first sheet and a rear end of the sensing pattern formed on the cut first sheet coincide with each other regardless of a cut position of the first sheet,
the sensing pattern includes a first sensing pattern and a second sensing pattern spaced apart from each other, and
a rear end of the first sensing pattern and a rear end of the second sensing pattern are both positioned at the rear end of the cut first sheet.

5. The method of claim 1, wherein the sensing pattern forming step is performed by a sensing pattern unit disposed between the source roll and the sensing pattern roll,
the sensing pattern unit includes a first cylindrical plate gravure rotary press that is disposed on a path of the first sheet moving from the source roll to the sensing pattern roll and rotates in pace with movement of the first sheet,
a plurality of trenches extending in parallel with each other is formed on an outer circumferential surface of the first cylindrical plate gravure rotary press,
each of the trenches is continuously formed without disconnection along an outer circumference of the first cylindrical plate gravure rotary press,
a conductive ink existing in the trenches is printed or coated on a side of the first sheet, and
a plurality of sensing patterns that is parallel with each other is continuously formed without disconnection by the trenches on the first sheet that has passed by the first cylindrical plate gravure rotary press in close contact with the first cylindrical plate gravure rotary press that is rotating.

6. The method of claim 5, wherein the trenches are formed in closed curve shapes with a start and an end connected on the outer circumferential surface of the first cylindrical plate gravure rotary press regardless of whether the first sheet is inclined in the longitudinal direction, and
the plurality of trenches is formed in parallel with same intervals in a width direction of the first sheet.

7. The method of claim 1, further comprising a pre-processing step of performing corona treatment on the first sheet unrolled from the source roll,
wherein a discharge treatment unit that performs corona treatment on the first sheet is disposed between the sensing pattern unit, which forms the sensing pattern, and the source roll, and
the sensing pattern unit forms the sensing pattern by printing or coating a conductive ink on the first sheet increased in hydrophilicity through the pre-processing step.

8. The method of claim 1, wherein the sensing pattern forming step forms the sensing pattern by printing a conductive ink on the first sheet using a sensing pattern unit,
a drying unit is disposed between the sensing pattern unit and the sensing pattern roll, and
the drying unit dries the sensing pattern formed on the first sheet before the first sheet is rolled on the sensing pattern roll.

9. The method of claim 1, wherein a marking line forming step of forming a marking line, which changes in color when being stained with excretion, on the first sheet is additionally provided, and
the marking line forming step attaches a litmus paper for testing acidity or alkalinity of the excretion to a side of the first sheet or prints or coats a litmus coloring matter to a side of the first sheet.

10. The method of claim 1, wherein the sensing pattern forming step forms the sensing pattern by printing or coating a conductive ink, which can transmit and receive the sensing signal, on a side of the first sheet, and
a litmus coloring matter for testing acidity or alkalinity of the excretion is contained in the conductive ink.

11. The method of claim 1, wherein a marking line forming step of forming a marking line, which changes in color when being stained with excretion, on the first sheet is additionally provided,
a guide guiding alignment of terminals formed at a terminal unit, which generates the sensing signal, and the sensing pattern is provided at the terminal unit, and
the marking line forming step forms the marking line to be spaced apart from the sensing pattern to fit to a gap between the guide and the terminals.

12. The method of claim 1, wherein the sensing pattern forming step is performed on a first sheet having a width larger than a width of an absorption product that holds the excretion,
the sensing pattern forming step forms the sensing pattern in every area corresponding to respective absorption products in a width direction of the first sheet, and
a slitting step of forming a plurality of first sheets having a width for manufacturing the absorption products by slitting one first sheet having a width larger than a width of the absorption products, using a slitting unit disposed between the sensing pattern unit, which performs the sensing pattern forming step, and the sensing pattern roll is provided.

13. The method of claim 1, wherein a sub-roll on which a second sheet made of a material passing the excretion is provided,
the sensing pattern roll and the sub-roll are installed to put the second sheet unrolled from the sub-roll onto the side on which the sensing pattern is printed or coated in the first sheet unrolled from the sensing pattern roll,
the first sheet and the second sheet are moved together to a bonding roller so that the second sheet is put on the side of the first sheet on which the sensing pattern is printed or coated, and
the second sheet is bonded to the side of the first sheet on which the sensing pattern is printed or coated, using the bonding roller.

14. The method of claim 13, wherein the first sheet and the second sheet bonded to each other and discharged from the bonding roller are cut to fit to a length of an absorption product that holds the excretion,
the first sheet and the second sheet cut to fit to the length of the absorption product are tailored to fit to the absorption product,
an absorber absorbing the excretion is stacked on a center of the second sheet tailored to fit to the absorption product and bonded to the first sheet, and
the absorber stacked on the center of the second sheet is covered with a third sheet made of a material that passes the excretion and then an edge of the third sheet is bonded to an edge of the second sheet.

15. The method of claim 1, wherein the first sheet and the second sheet are combined in the same size,
the second sheet is put on the first sheet with the sensing pattern therebetween, and
when the first sheet and the second sheet combined with each other are cut, a rear end of the sensing pattern coincides with a rear end of the first sheet and a rear end of the second sheet.
